# EUROPEAN PATENT SPECIFICATION

(11) **EP 3 576 826 B1**
(45) Date of publication and mention of the grant of the patent: **25.09.2024**
(21) Application number: 17718690.5
(22) Date of filing: 06.04.2017
(51) Int. Cl.: A61M 25/00, A61M 25/04

(54) **CATHETER SYSTEM FOR CONTINUOUS IRRIGATION**
KATHETERSYSTEM ZUR KONTINUIERLICHEN SPÜLUNG
SYSTÈME DE CATHÉTER POUR IRRIGATION CONTINUE

(30) Priority: 05.02.2017 US 201762454829 P
(43) Date of publication of application: 11.12.2019
(73) Proprietor: CIC Fund Securitisation S.A., 1253 Luxembourg (LU)
(72) Inventor: MCINTYRE, Matthew G., Theodore, AL 36592 (US)
(74) Representative: Aronova
(86) International application number: PCT/US2017/026450
(87) International publication number: WO 2018/144045

(56) References cited:
- FR-A- 1 280 481
- US-A- 3 981 299
- US-A- 5 269 755
- US-A- 5 417 657
- US-A1- 2016 367 747

## Description

### FIELD

The present invention pertains to a catheter, and more particularly, to intra-urethral or indwelling catheters capable of effluxing fluids.

### BACKGROUND

The traditional Foley-type catheter is well known in the art and comprises an inflatable balloon disposed within the patient's bladder and a discharge tube extending through the urethra to the exterior. The Foley-type catheter provides passive urinary drainage, and the ability to clamp the catheter closed at a location exterior of the patient.

Urethral catheters, such as Foley-catheters, are used to drain urine from the bladder. A urinary tract infection (also called "UTI") is an infection in the urinary system, which includes the bladder and kidneys. When a urinary catheter is inserted into the bladder, germs can migrate along the catheter and cause an infection in the bladder or kidney; resulting in a catheter-associated urinary tract infection (or "CAUTI"). CAUTis are the most common of hospital-acquired infections. In fact, 40% of all nosocomial infections and over 100,000 admissions to hospital within the USA annually are attributable to CAUTIs. ¹ Outcomes associated with CAUTIs include bacteremia and sepsis. While morbidity that is attributable to a single episode of catheterization is limited, the high frequency of catheter use (around 25% of hospitalized patients) means that the cumulative burden of CAUTIs on patients and hospitals is substantial.²
¹ D. Cardo et al. National Nosocomial Infections Surveillance (NNIS) System Report, data summary from January 1992 through June 2004, issued October 2004. Am. J. Infect. Control, 32 (2004), pp. 470-485.
² Lo, E. et al. (2008). Strategies to Prevent Catheter- Associated Urinary Tract Infections in Acute Care Hospitals. Infection Control and Hospital Epidemiology, 29(S1), S41-S50. doi: 10. 1086/591066

When sterile urinary catheters are inserted into the bladder, components in urine, blood, or surrounding tissue, such as polysaccharides, ions, and glycoproteins, are deposited on the surface of the device allowing the formation of biofilms. Biofilms are highly structured and actively growing bacterial communities that consist of multiple bacterial layers protected by a thick exopolysaccharide layer³. Biofilms are resistant to antibiotics/antimicrobials due to the fact that these agents cannot penetrate sufficiently through the exopolysaccharide layer.
³ Tenke, P.; Koves, B.; Nagy, K.; Hultgren, S.J.; Mendling, W.; Wullt, B.; Grabe, M.; Wagenlehner, F.M.; Cek, M.; Pickard, R.; et al. Update on biofilm infections in the urinary tract. World J. Urol. 2012, 30, 51-57.

According to Centers for Disease Control and Prevention (CDC), there was no change in overall catheter-associated urinary tract infections (CAUTI) rates between 2009 and 2014. (see https://www.cdc.gov/hai/surveillance/). This is not surprising, as while a variety of approaches for prevention of biofilm formation include the use of biocoatings, impregnating materials with antibiotics, antimicrobials or other materials as well as catheters capable of eluting antibiotics and/or antimicrobials have been used, none have been fully effective. Further, one of the major complications associated with antibiotic based coatings is the development of resistance. For example, one approach has been to attach active biocides such as antibiotics to biomaterial surfaces, or to impregnate them into the biomaterial itself by coating device surfaces or impregnating device surfaces with antibiotics such as ciprofloxacin, gentamicin, norfloxacin, and nitrofurazone. When used in clinical studies, the uncontrolled release profiles of the drugs resulted in the elution of initial high local concentrations that may initially damage the cells followed by concentrations that are not inhibitory. ⁴ By not killing all of the bacteria effectively, any subsequent infection will be more difficult to eradicate due to the development of resistance.
⁴ Walder, B.; Pittet, D.; Tramer, M.R. Prevention of bloodstream infections with central venous catheters treated with anti-infective agents depends on catheter type and insertion time: Evidence from a meta-analysis. Infect. Control Hosp. Epidemiol. 2002, 23, 748-756.

Looking at the physiology of the urethra, UTIs are generally avoided because the act of urination (voiding) flushes everything, including bacteria. Further, there are glands in urethra that secretes protecting mucus. Several drug eluting urinary catheters are known in the prior art. Drug-eluting urinary catheters generally consist of three parts - the catheter tube, a polymer coating that binds the drug to the tube and releases the drug. The drug is slowly and continuously released into the bladder or along urethra; however, there is no continual washing of the periurethral space, where bacteria adhere, form biofilms and result in bacterial infections.

It would therefore be useful to magnify the effect of the glands in the urethra that protect from infection in the context of catheters.

FR 1 280 481 A discloses a catheter applicable, in particular, in urology. Its purpose is to allow local medications to be administered by spreading them, preferably slowly. It is characterized by the fact that the catheter is constituted by a conduit ending in a cannula to which is attached a container opening into a capacity closed by a dialytic membrane, a balloon inflatable with the liquid to be injected, which balloon prevents the exit of the catheter from the capacity into which it has been introduced.

US 2016/367747 A1 relates to a catheter, in particular a balloon catheter, having a fluid-carrying element, such as a drainage tube, with an inner lumen for carrying away body fluid, and having an expansion element, such as a balloon of variable cross section, for fixing the catheter in a body cavity. The fluid-carrying element has a wall portion of an open-pore material, which is in fluidic communication with the inner lumen and through which the body fluid can be aspirated into the inner lumen by applying a negative pressure to a proximal end of the fluid-carrying element.

US 3,981,299 A discloses an urethral catheter construction having a third tubular extension or finger which will permit injections of antibiotics or an anesthetic onto the outer wall of the catheter through a very thin porous rubber outer membrane.

US 5,417,657 A discloses a no-sepsis urinary catheter, comprising three lumens, each in fluid communication with a drainage tip for receiving urine from the bladder, a retention balloon for retaining the catheter within the bladder, and a microporous bacteriostasis balloon for the diffusion by osmosis of a pharmaceutical agent for the killing and prevention of bacteria growth within and around the bladder to preclude the development of sepsis therein.

US 5,269,755 A discloses a catheter having an outer sheath made of a porous polymer material such as expanded polytetrafluoroethylene (ePTFE). The sheath has a porosity to allow anti-microbial or anti-bacterial medicament or other medicaments or liquids to pass through the sheath. A medicament lumen is provided passing through the elongated tube of the catheter from the catheter's proximal or out of patient end to openings along the catheter tube wall. Medicament is passed from the proximal end of the catheter through the medicament lumen and expelled through the openings into the area between the sheath and the catheter's main tube. There, the medicament or other liquid passes through the sheath into contact with the patient's body lumen. In the preferred embodi¬ ment, the catheter is a Foley urinary catheter.

### BRIEF SUMMARY OF THE INVENTION

The present invention is defined in independent claim 1. It is one object of the present invention to provide an indwelling urinary catheter system having (1) an elon¬ gated tubular catheter body having a distal end and a proximal end; (2) at least one sleeve portion constructed substantially out of a semipermeable membrane surrounding at least one portion of the catheter body; (3) at least one lumen to instill fluid into the catheter body; and (4) a means to continuously efflux the instilled fluid through the semi¬ permeable membrane of at least one sleeve resulting in the circumferential egress of fluid out of the semipermeable membrane around the catheter body. The catheter may further include a drainage lumen extending through the catheter body from just short of the distal end to the proximal end and an opening or eyelet in the catheter body just short of the distal end of the catheter body to permit urine to drain from a patient's bladder into the drainage lumen. The catheter body is disposed within the urethra of the patient and a retaining mechanism, such as an inflatable balloon, is disposed within the patient's blad¬ der to retain the catheter in position. The fluid instilled into the catheter body and effluxed from the sleeve portion(s) may include, but is not limited to, antiseptics, antibiotics or an¬ timicrobials, and/or combinations thereof to prevent biofilm formation on the exterior sur¬ face of the catheter body. The fluid may also include certain therapeutic agents used in intravesical therapy, such as immunotherapy agents or chemotherapeutic agents. The fluid may also include agents for patient comfort, such as antispasmodics and pain medi¬ cines. All such agents can be effluxed directly into the bladder through the semipermeable sleeve portion around the catheter tip placed within the bladder.

It is another object of the present invention to provide different embodiments of the urinary catheter system that match the particular anatomical characteristics of a patient with respect to male or female anatomy. For example, a retention collar may be positioned on the catheter body for female patients or a space may be provided for the prostate for male patients.

### BRIEF DESCRIPTION OF THE DRAWINGS

**Figure 1.** Figure 1 is a cross section view of a traditional catheter for insertion into the bladder.
**Figure 2.** Figure 2 is a front perspective view of a traditional 2-way urinary catheter.
**Figure 3.** Figure 3 is a front perspective view of a traditional 3-way urinary catheter with a cutaway cross section of the catheter body.
**Figure 4A.** Figure 4A is a front perspective view of one embodiment of the urinary catheter of the present invention with a cutaway cross section of the catheter body.
**Figure 4B.** Figure 4B is a front perspective view of one embodiment of the urinary catheter of the present invention with a cutaway cross section of the sleeve section.
**Figure 5A.** Figure 5A is a front perspective view of an alternative embodiment of the urinary catheter of the present invention with a cutaway cross section of the catheter body.
**Figure 5B.** Figure 5B is a front perspective view of an alternative embodiment of the urinary catheter of the present invention with a cutaway cross section of the sleeve.
**Figure 6A.** Figure 6A is a front perspective view of an alternative embodiment of the urinary catheter of the present invention with a cutaway cross section of the catheter body.
**Figure 6B.** Figure 6B is a front perspective view of an alternative embodiment of the urinary catheter of the present invention with a cutaway cross section of the sleeve.
**Figure 7A.** Figure 7A is a cross section view of the placement of a catheter in a male.
**Figure 7B.** Figure 7B is a cross section view of the placement of a catheter in a female.
**Figure 8A.** Figure 8A is a front perspective view of one embodiment of the present invention for use in female patients.
**Figure 8B.** Figure 8B is a front perspective view of one embodiment of the present invention for use in female patients with a cutaway cross section of the sleeve.
**Figure 9A.** Figure 9A is a front perspective view of one embodiment of the present invention for use in male patients.
**Figure 9B.** Figure 9B is a front perspective view of one embodiment of the present invention for use in male patients with a cutaway cross section of the sleeve.
**Figure 10A.** Figure 10A is a front perspective view of one embodiment of the present invention with a couvelaire tip.
**Figure 10B.** Figure 10B is a front perspective view of one embodiment of the present invention with a dufour tip.
**Figure 10C.** Figure 10C is a front perspective view of one embodiment of the present invention with a coude tip.
**Figure 11A.** Figure 11A is a front perspective view of an alternative embodiment of the present invention with a couvelaire tip.
**Figure 11B.** Figure 11B is a front perspective view of an alternative embodiment of the present invention with a dufour tip.
**Figure 11C.** Figure 11C is a front perspective view of an alternative embodiment of the present invention with a coude tip.
**Figure 12A.** Figure 12A is a front perspective view of an alternative embodiment of the present invention with a couvelaire tip.
**Figure 12B.** Figure 12B is a front perspective view of an alternative embodiment of the present invention with a dufour tip.
**Figure 12C.** Figure 12C is a front perspective view of an alternative embodiment of the present invention with a coude tip.

### DETAILED DESCRIPTION

For the purposes of the present invention, the term "semipermeable" is intended to encompass not only those materials that are semipermeable by their nature (i.e. those that allow certain substances to pass through it while not allowing other materials to pass through it) but materials that may be made semipermeable by creating pores of a predetermined size that would allow certain substances to pass through it while not allowing other materials to pass through it.

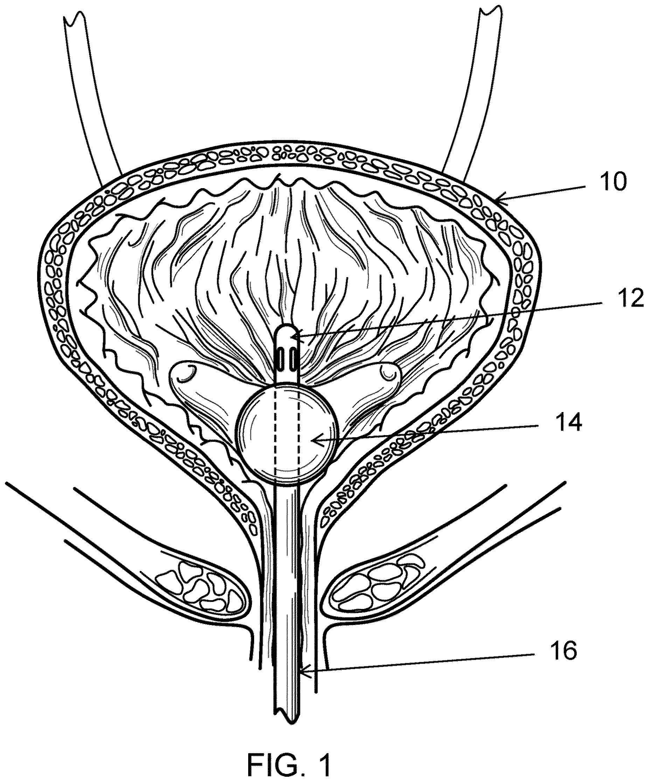

Turning to the drawings, there shown in Fig. 1 is a traditional catheter for insertion into a cavity, duct, or a vessel to permit injection or withdrawal of fluids into or from the cavity, duct, or vessel, or to establish patency of a passageway. For example, the catheter body **16** may be inserted through a patient's urethra and into the patient's bladder **10** for draining urine from the bladder and/or instilling fluid into the bladder through slots in the tip **12** of the catheter. A retaining device, such as the balloon **14,** is used to maintain placement of the catheter in the bladder.

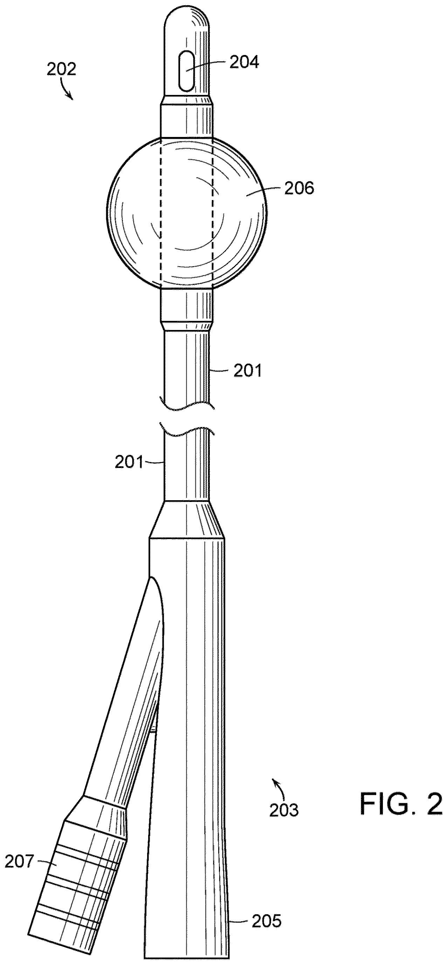

Turning to Fig. 2, a traditional 2-way urinary catheter is represented with a catheter body **201** having a distal end **202** and a proximal end **203** with the catheter body **201** connecting an opening or eyelet **204** at the distal end **202** to a drainage lumen **205** at the proximal end **203** of the catheter body **201** through which fluid may flow into the drainage lumen **205** when the catheter is used to drain fluid from the bladder. An inflatable tube section **206** with an inflation lumen **207** extends along the length of the catheter body **201** and communicates with the inflatable tube section **206.** Inflation fluid, such as distilled water, is passed through inflation lumen **207** into the tube section **206** to inflate the tube section **206,** and the inflation fluid is withdrawn from the tube section **206** into and through the inflation lumen **207** when it is desired to deflate the tube section **206.**

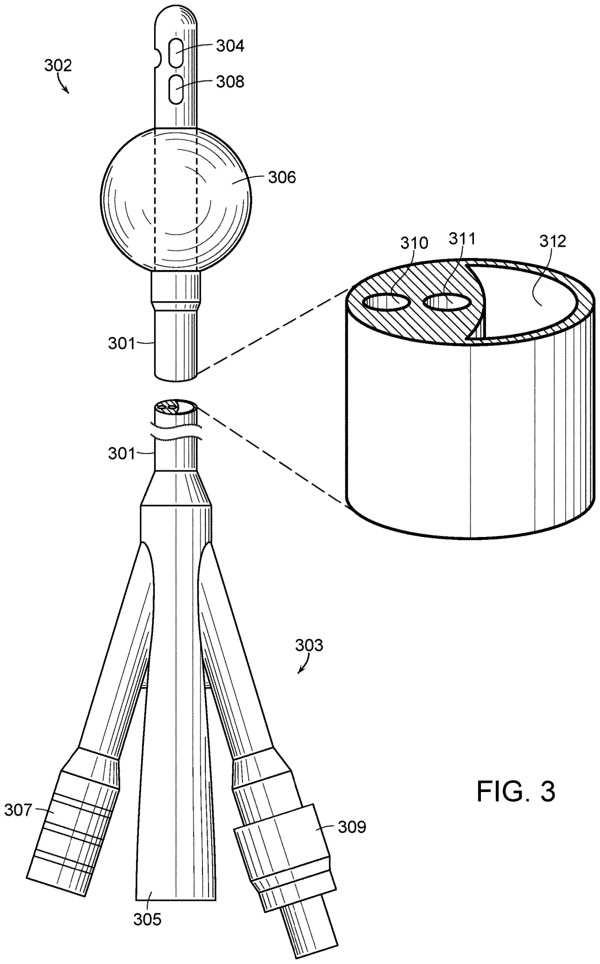

Turning to Fig. 3, a traditional 3-way urinary catheter is represented that is essentially the same as the catheter shown in Fig. 2, except it includes an instillation lumen **309** that extends from the catheter body **301** at the proximal end **303.** The fluid instilled into the catheter body **301** is passed through tube **311** in the catheter body **301** and into the bladder through the opening or eyelet **304** and then the fluid is subsequently drained through the opening or eyelet **308** through tube **312** in the catheter body **301** and out the drainage lumen **305.** As shown in the cross section, the fluid instilled into the catheter body **301** passes through tube **311** in the catheter body. Inflation fluid is passed through inflation lumen **307** and through tube **310** to inflate the tube section **306.** Fluid that is drained through eyelet **308** at the distal end **302** passes through tube **312** and out the drainage lumen **305.**

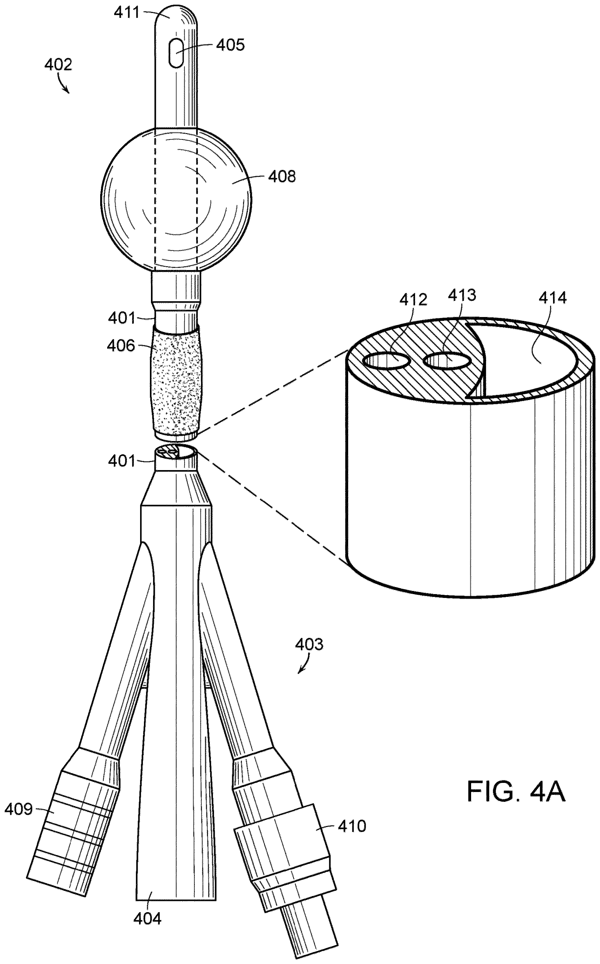

Referring to Fig. 4A, the catheter of the present invention includes an elongated tubular catheter body **401** having a distal end **402** and a proximal end **403.** A drainage lumen **404** extends through tube **414** in the catheter body **401** from the distal end **402** to the proximal end **403.** The drainage lumen **404** communicates with an opening or eyelet **405** in the catheter body **401** at the distal end **402** of the catheter body **401** through which the fluid may flow into the drainage lumen **404** when the catheter is used to drain a fluid from a cavity, duct, or vessel (e.g., draining urine from a person's bladder). A sleeve portion **406** constructed from a semipermeable membrane is formed over the catheter body **401.** An instillation lumen **410** extends from the catheter body **401** at the proximal end **403.** The instillation lumen **410** connects with the sleeve portion **406** using tube **413** that runs through the length of the catheter body **401.** The fluid instilled into the catheter body **401** through the tube **413** is continuously effluxed from the sleeve portion **406** through the semipermeable membrane in a circumferential controlled delivery to continuously irrigate the periurethral space and the catheter body **401** to prevent formation of biofilm and further ensuing bacterial infection. The fluid may include, but is not limited to, antiseptics, antibiotics or antimicrobials and/or combinations thereof to prevent biofilm formation on the exterior surface of the catheter body. Inflation fluid is passed through inflation lumen **409** and through tube **412** in the catheter body **401** to inflate the tube section **408.**

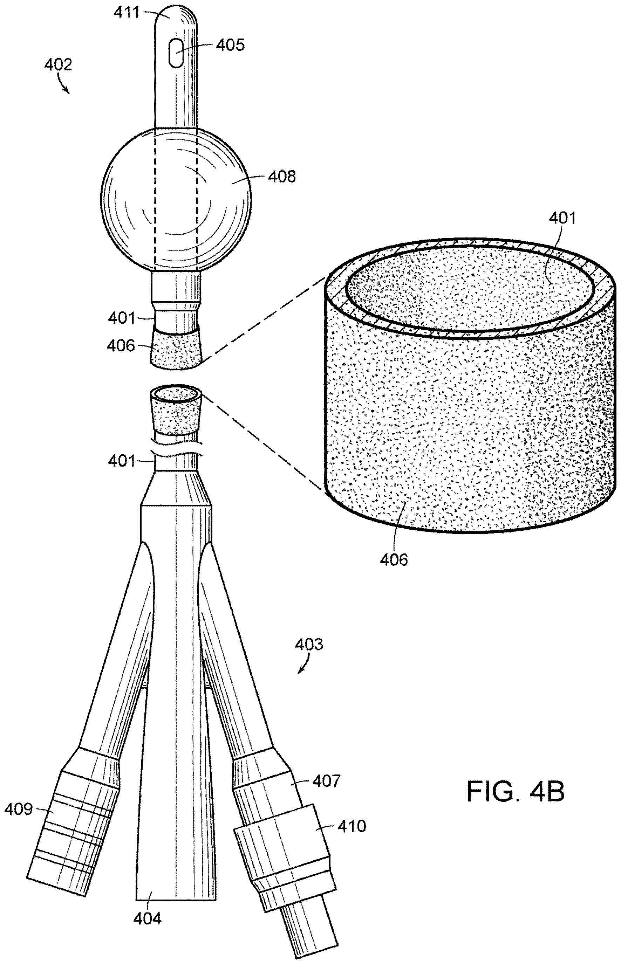

Turning to Fig. 4B, a cross section cutaway of the sleeve portion **406** illustrates that the sleeve circumferentially surrounds the catheter body **401.** In the preferred embodiment, the sleeve **406** is manufactured as a continuous part over the catheter body **401.** It may be secured to the catheter body **401** using methods known in the art such as adhesive attachment or heat press melting. Additionally, the sleeve **406** is preferably constructed from a non-elastic material to allow the effluxed fluid to irrigate the periurethral space without putting pressure on the urethra. In the preferred embodiment, the fluid effluxed from the sleeve **406** exits through the urethral opening and may be collected by a sponge or padded surface. Ideally around 300-500mL of fluid a day would be effluxed resulting in a collection rate in the sponge or padded surface of about 20ccs per hour. This is manageable in a hospital care setting with intermittent replacement of the sponge or padded surface.

Referring to Fig.4A, in the preferred embodiment a retaining mechanism near the distal end **402** of the catheter body **401** is generally an inflatable tube section **408** with an inflation lumen **409** that extends the length of the catheter body **401** through tube **412** and communicates with the inflatable tube section **408.** Inflation fluid, such as distilled water, is passed through inflation lumen **409** into the tube section **408** to inflate the tube section **408,** and the inflation fluid is withdrawn from the tube section **408** into and through the inflation lumen **409** when it is desired to deflate the tube section **408.** When the inflatable tube section **408** is not inflated, it lies substantially parallel along the central axis of the catheter body **401,** forming a cylinder having a diameter that substantially matches the outer diameter of the catheter body **401.**

The fluid instilled into the catheter body **401** and effluxed out of the semipermeable membrane sleeve **406** of the catheter body may be pushed through the device using various mechanisms, including but not limited to, a pressure and flow regulating valve to control rate of flow for a specific fluid at a specific pressure that is installed at the effluxing instillation lumen **410** or using a pump tension device, such as a plastic ball that is blown up and then pushes fluid out at a constant rate. It is also contemplated that an intravenous (IV) pump operating at a continuous rate may also be used to move fluid through the instillation lumen **410** and out of the semipermeable membrane of the sleeve portion **406.** Again, the rate would be predetermined based on the semipermeable membrane material as well as the molecular weight cut off (MWCO) of the agent instilled into the catheter and effluxed through the semipermeable membrane to ensure that the agent is being pushed with sufficient pressure and at a sufficient rate to effectively continuously wash the periurethral space around the catheter body **401.**

It is further contemplated that a drug eluting portion could be located within the tip **411** of catheter body **401** that goes into the bladder that could be used to deliver drugs to the bladder itself, such as an antispasmodic, pain medicines, antibiotics, antiseptics, antimicrobials and combinations thereof.

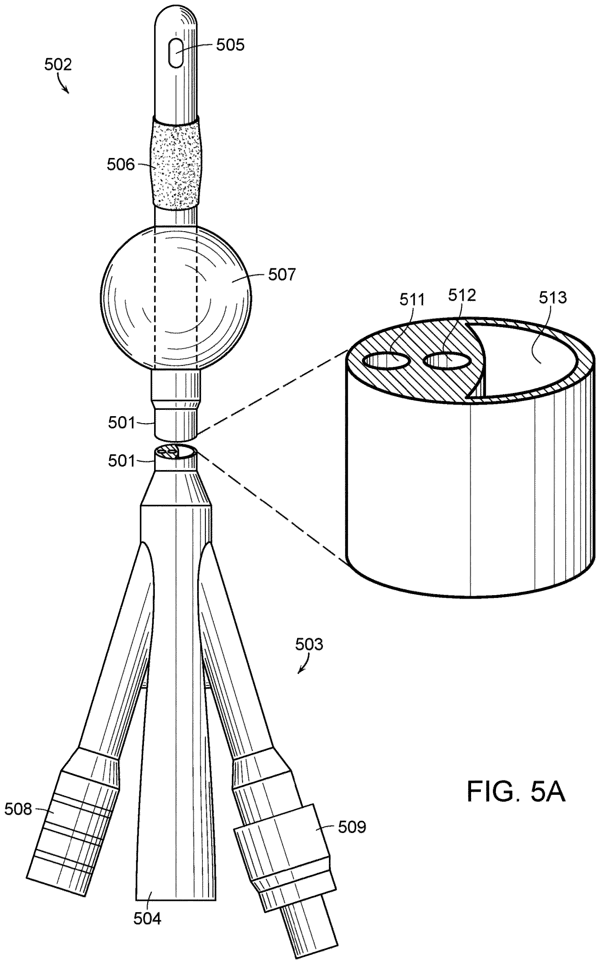

Turning to Fig. 5A, an alternative embodiment of the present invention is represented with an elongated tubular catheter body **501** having a distal end **502** and a proximal end **503.** A drainage lumen **504** extends through tube **513** in the catheter body **501** from the distal end **502** to the proximal end **503,** and the drainage lumen **503** communicates with an opening or eyelet **505** in the catheter body **501** at the distal end **502** of the catheter body **501** through which the fluid may flow into the drainage lumen **504** when the catheter is used to drain a fluid from a cavity, duct, or vessel (e.g., draining urine from a person's bladder). The retaining mechanism in this example is an inflatable tube section **507** with an inflation lumen **508** that extends though the length of the catheter body **501** though tube **511** and communicates with the inflatable tube section **507.** Inflation fluid, such as distilled water, is passed through inflation lumen **508** into the tube section **507** to inflate the tube section **507,** and the inflation fluid is withdrawn from the tube section **507** into and through the inflation lumen **508** when it is desired to deflate the tube section **507.** When the inflatable tube section **507** is not inflated, it lies substantially parallel along the central axis of the catheter body **501,** forming a cylinder having a diameter that substantially matches the outer diameter of the catheter body **501.**

A sleeve portion **506** constructed from a semipermeable membrane is formed over the catheter body **501** above the tube section **507.** An instillation lumen **509** extends from the catheter body **501** at the proximal end **504.** The instillation lumen **509** connects with the sleeve portion **506** using tube **512** that runs through the length of the catheter body **501.** The fluid instilled into the catheter body **501** through the tube is continuously effluxed from the sleeve portion **506** through the semipermeable membrane and into the bladder.

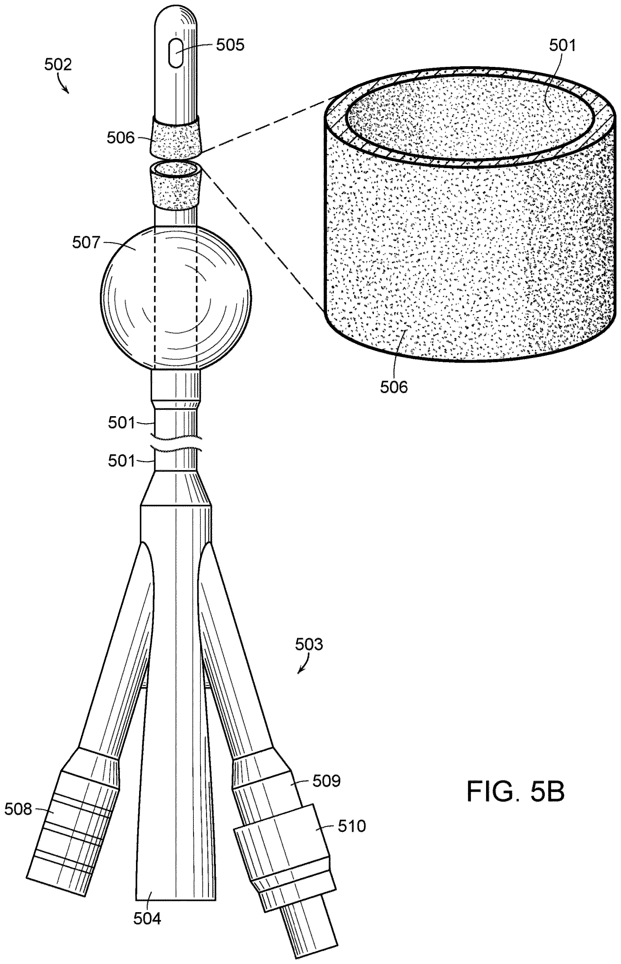

Turning to Fig. 5B, a cross section cutaway of the sleeve portion **506** illustrates that the sleeve circumferentially surrounds the catheter body **501.** In the preferred embodiment, the sleeve **506** is manufactured as a continuous part over the catheter body **501.** It may be secured to the catheter body **501** using methods known in the art such as adhesive attachment or heat press melting. The fluid effluxed through the sleeve **506** includes, but is not limited to, certain therapeutic agents used in intravesical therapy, such as immunotherapy agents or chemotherapeutic agents, as well as antispasmodic agents and numbing agents such as lidocaine. The semipermeable membrane of the sleeve **506** allows certain substances to pass through it but not others, such as allowing fluids to efflux out of the sleeve **506** but not allowing bacteria or other contaminants into the sleeve **506.** The semipermeable membrane also allows the use of a small amount of fluid everywhere circumferentially along the length of the catheter body portion in the bladder as well as into the bladder space. The pore size of the semipermeable membrane is predetermined based on the agent instilled into the catheter and effluxed from the semipermeable membrane to ensure that the agent may pass through the semipermeable membrane of the sleeve **506** and may be effluxed with sufficient pressure and at a sufficient rate to effectively continuously wash the bladder with the fluid. This method is a superior mechanism to deliver therapies such as antispasmodic agents and numbing agents than an instillation performed using a traditional catheter. With a traditional catheter, instillations are performed on an intermittent basis wherein the medicine is delivered through a single lumen catheter and then removed. The patient then voids the bladder to remove the medicine. The present invention allows the medicine to be slowly effluxed into the bladder at a continuous rate. This is especially useful after transurethral surgery on a patient. The catheter of the present invention can be placed shortly after surgery so that a drug, such as an antispasmodic or pain medication, may be effluxed from the sleeve **506** for the next four to six hours, resulting in steady patient pain and discomfort management.

The fluid instilled into the catheter body and effluxed out of the semipermeable membrane of the sleeve portion **506** over the catheter body **501** and into the bladder may be pushed through the device using various mechanisms, including but not limited to, a pressure and flow regulating valve to control rate of flow for a specific fluid at a specific pressure that is installed at the effluxing instillation lumen port **510** or using a pump tension device, such as a plastic ball that is blown up and it then pushes fluid out at a constant rate. It is also contemplated that an intravenous (IV) pump operating at a continuous rate may also be used to move fluid through the instillation lumen and out of the semipermeable membrane of the sleeve portion **506.** Again, the rate would be predetermined based on the agent instilled into the catheter and effluxed from the semipermeable membrane to ensure that the agent is being pushed with sufficient pressure and at a sufficient rate to effectively continuously wash the bladder space.

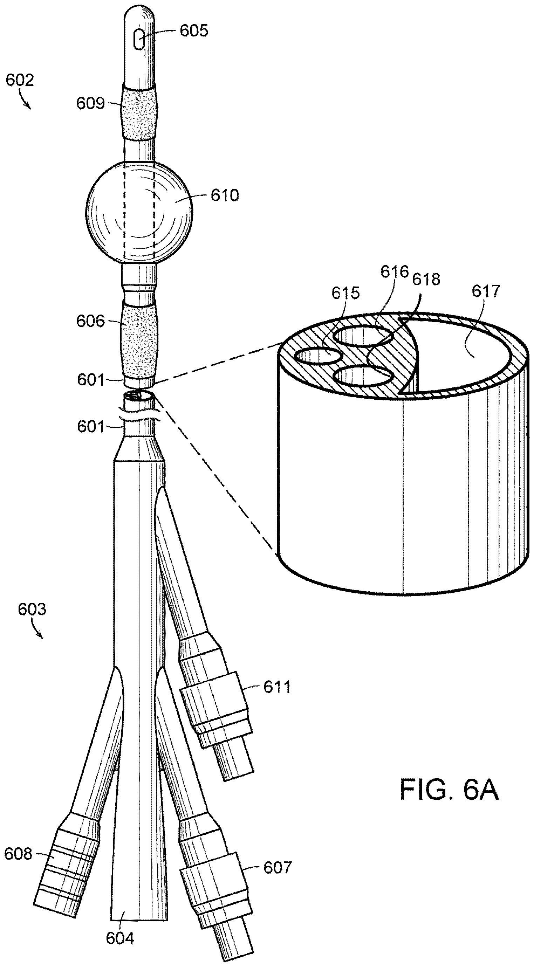

Turning to Figs. 6A-B, another embodiment of the present invention uses both sleeve portions of Figs. 4-5. This results in a 4 way catheter capable of both effluxing fluid to continuously irrigate the periurethral space as well as effluxing fluid to continuously wash the bladder space.

As shown in Fig. 6A an elongated tubular catheter body **601** having a distal end **602** and a proximal end **603.** A drainage lumen **604** extends through tube **617** in the catheter body **601** from the distal end **602** to the proximal end **603,** and the drainage lumen **604** communicates with an opening or eyelet **605** in the catheter body **601** at the distal end **602** of the catheter body **601** through which the fluid may flow into the drainage lumen **604** when the catheter is used to drain a fluid from a cavity, duct, or vessel (e.g., draining urine from a person's bladder). A first sleeve portion **606** constructed from a semipermeable membrane is formed over the catheter body **601.** An instillation lumen **607** extends from the catheter body **601** at the distal end **602.** The instillation lumen **607** connects with the first sleeve portion **606** using tube **616** that runs through the length of the catheter body **601.** The fluid instilled into the catheter body **601** through the tube is continuously effluxed from the sleeve portion **606** through the semipermeable membrane in a circumferential controlled delivery to continuously irrigate the periurethral space and the catheter body **601** to prevent formation of biofilm and further ensuing bacterial infection. The fluid may include, but is not limited to, antiseptics, antibiotics or antimicrobials and/or combinations thereof to prevent biofilm formation on the exterior surface of the catheter body.

A second sleeve portion **609** constructed from a semipermeable membrane is formed over the catheter body **601** above the tube section **610.** An instillation lumen **611** extends from the catheter body **601** at the distal end **602.** The instillation lumen **611** connects with the sleeve portion **609** using tube **618** that runs through the length of the catheter body **601** The fluid instilled into the catheter body **601** through the tube **618** is continuously effluxed from the sleeve portion **609** through the semipermeable membrane and into the bladder itself.

The fluid effluxed through the sleeve **609** includes, but is not limited to, certain therapeutic agents used in intravesical therapy such as immunotherapy agents or chemotherapeutic agents, antispasmodic agents and numbing agents, such as lidocaine.

The fluid instilled into the catheter body and effluxed out of the semipermeable membrane of the sleeve portions **606** and **609** may be pushed through the device using various mechanisms, including but not limited to, pressure and flow regulating valves to control rate of flow for a specific fluid at a specific pressure that is installed at the effluxing instillation lumen ports **607** and **611,** or using a pump tension device, such as a plastic ball that you blow up and it then pushes fluid out at a constant rate. It is also contemplated that an intravenous (IV) pump operating at a continuous rate may also be used to move fluid through the instillation lumens **607** and **611** and out of the semipermeable membrane of the sleeve portions **606** and **609,** respectively. Again, the rate would be predetermined based on the agent instilled into the catheter and effluxed from the semipermeable membrane to ensure that the agent is being pushed with sufficient pressure and at a sufficient rate to effectively continuously wash the periurethral and bladder spaces.

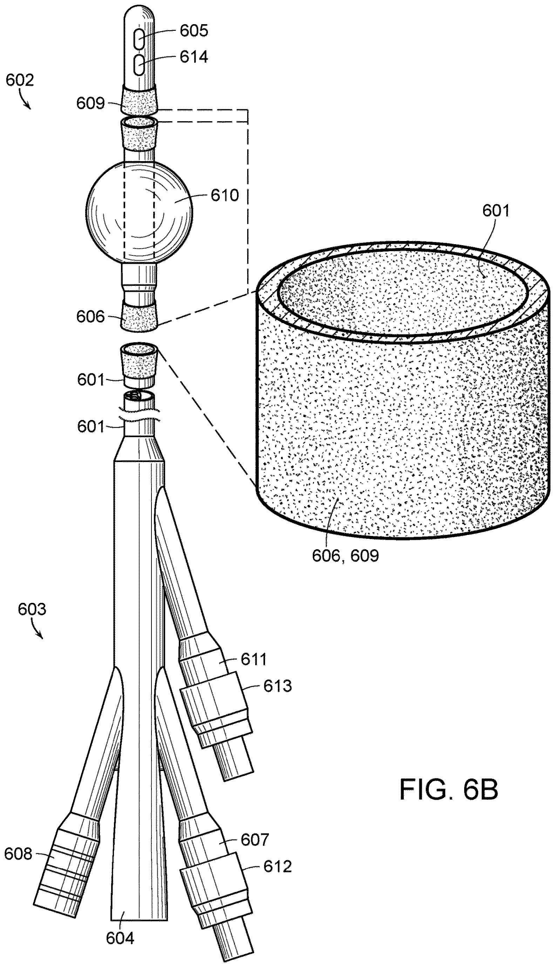

Turning to Fig. 6B, a cross section cutaway of the sleeve portions **606** and **609** illustrates that the sleeve circumferentially surrounds the catheter body **601.** In the preferred embodiment, the sleeve portions **606** and **609** are manufactured as continuous parts over the catheter body **601.** They may be secured to the catheter body **601** using methods known in the art such as adhesive attachment or heat press melting.

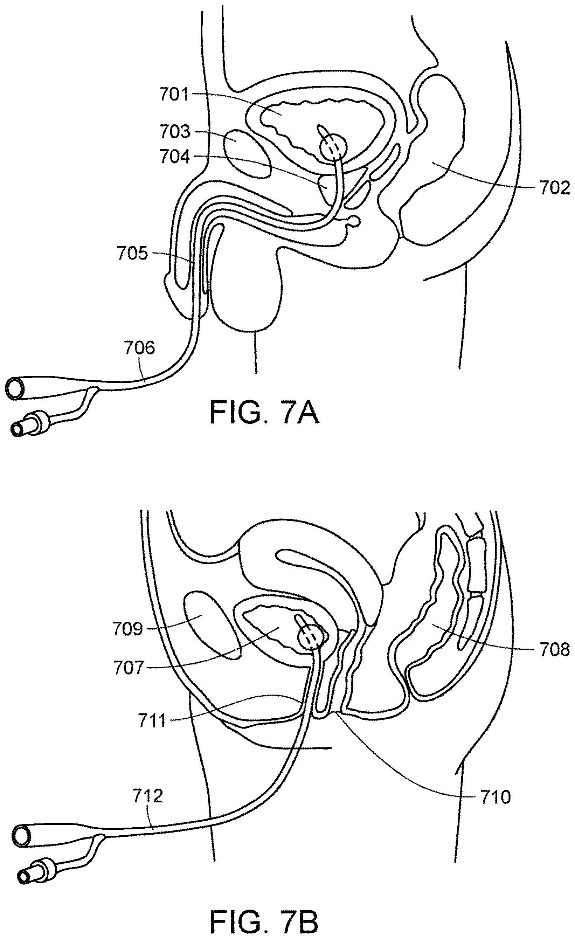

Turning to Fig. 7A-B, the differences in anatomy for the placement of a urinary catheter are shown. The male anatomy of Fig. 7A results in a larger portion of the catheter body in the periurethral space than the female counterpart. Fig. 7A shows the bladder **701,** rectum **702,** pubic bone **703,** prostate **704,** urethra **705** and the catheter **706.** The catheter **706** must also be fed past the prostate **704** in males before it can be retained in the bladder **701.** The female anatomy of Fig. 7B results in a shorter portion of the catheter body needed to fill the periurethral space. Fig. 7B shows the bladder **707,** rectum **708,** pubic bone **709,** vagina **710,** urethra **711** and catheter **712.**

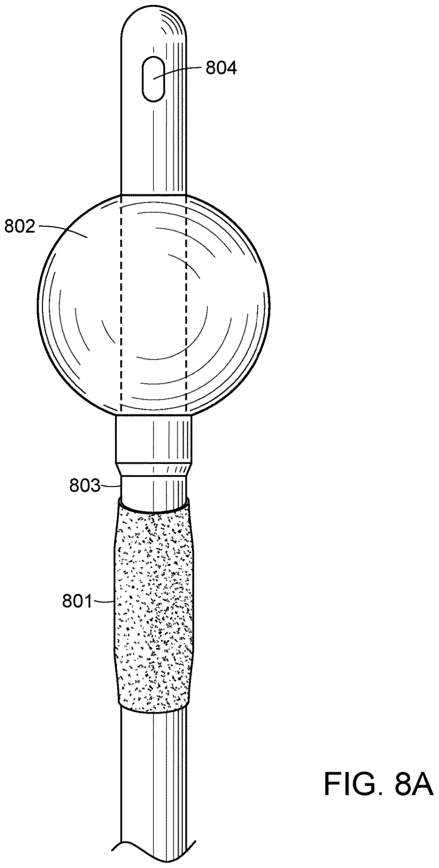

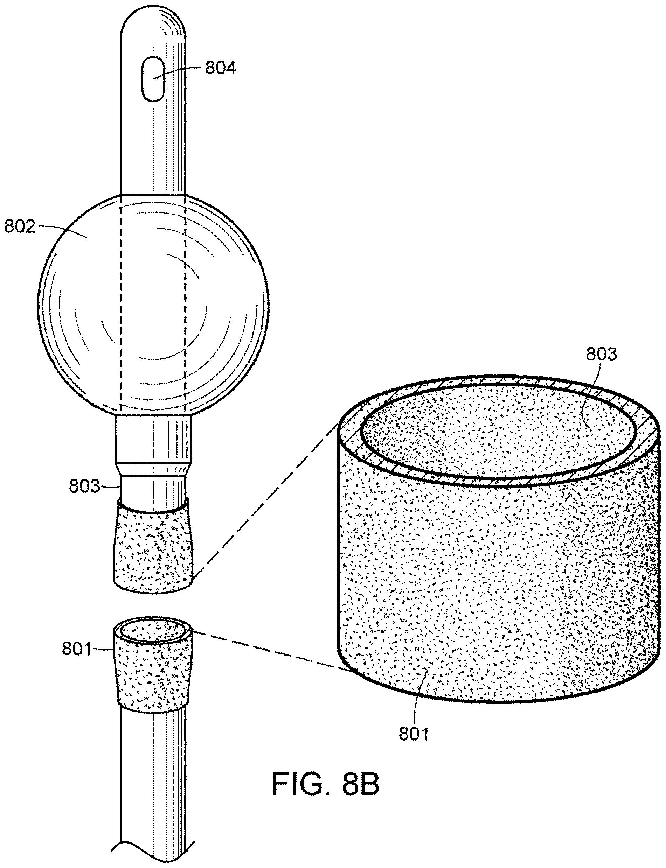

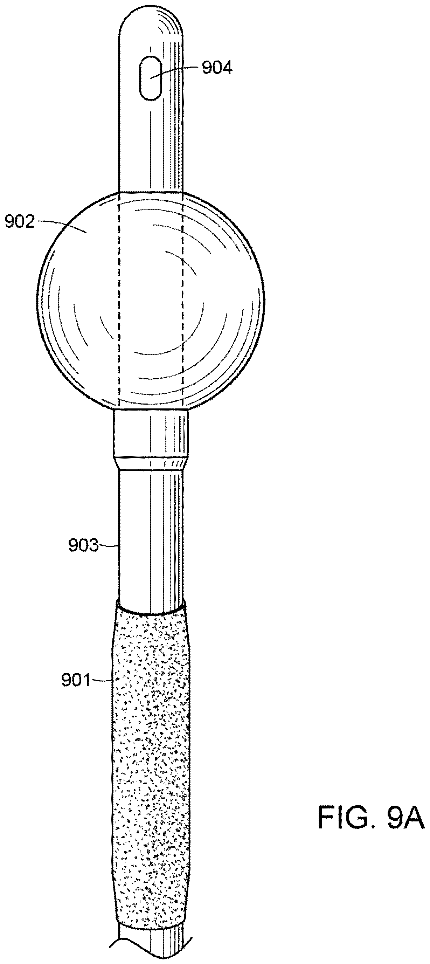

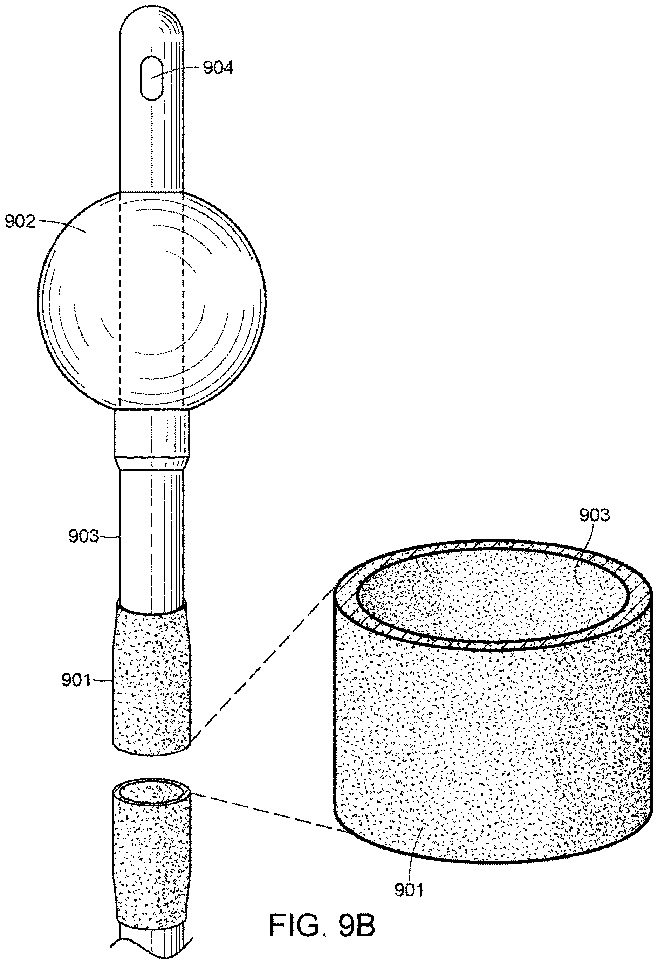

Taking these anatomical differences into consideration, Fig. 8A-B shows the distal end of the catheter of Fig. 4 as used for female anatomy whereas Fig. 9A-B shows the distal end of the catheter of Fig. 5 as used for male anatomy. The sleeve portion **801** of Fig. 8A-B is shorter than the sleeve portion **901** of Fig. 9A-B. Additionally, there is a larger space **903** between the sleeve portion **901** and the inflatable portion **902** than the space **803** between the sleeve portion **801** and the inflatable portion **802,** which accommodates placement of the catheter in the presence of the prostate.

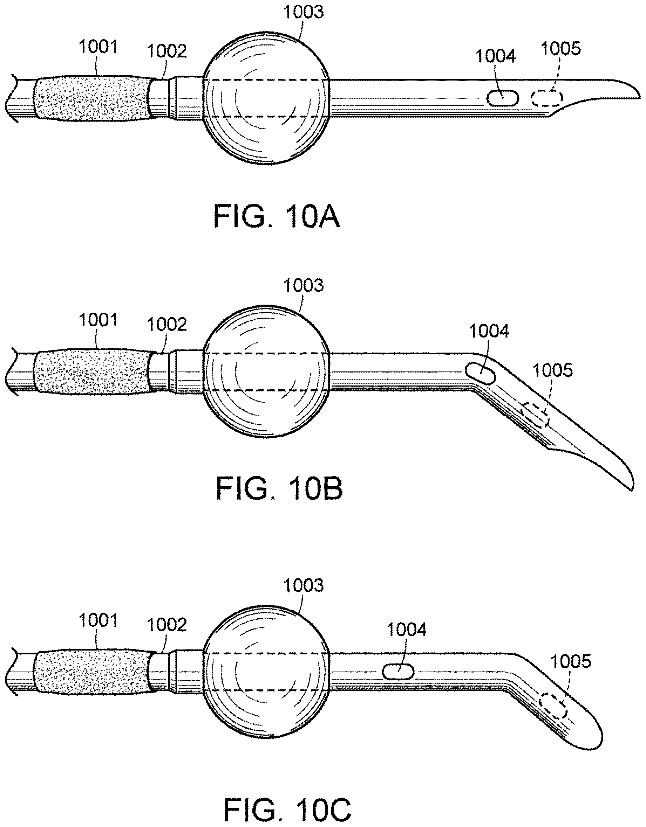

As shown in Figs. 10A-C, one embodiment of the invention shown in Figs. 4A-B with sleeve portion **1001,** catheter body **1002,** retaining device **1003,** drainage eyelet **1004** and alternative instillation eyelet **1005** may have various shapes to the end that is inserted into the bladder. For example, Fig. 10 A shows a couvelaire tip, Fig. 10B shows a dufour tip and Fig. 10C shows a coude tip.

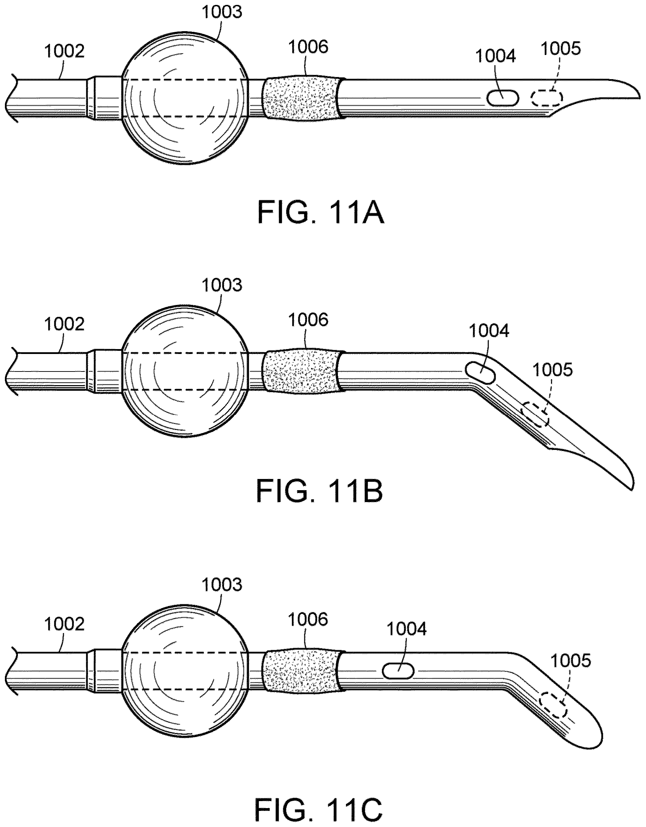

As shown in Figs. 11A-C, one embodiment of the invention shown in Figs. 5A-B with sleeve portion **1006,** catheter body **1002,** retaining device **1003,** drainage eyelet **1004** and alternative instillation eyelet **1005** may have various shapes to the end that is inserted into the bladder. For example, Fig. 11 A shows a couvelaire tip, Fig. 11B shows a dufour tip and Fig. 11C shows a coude tip.

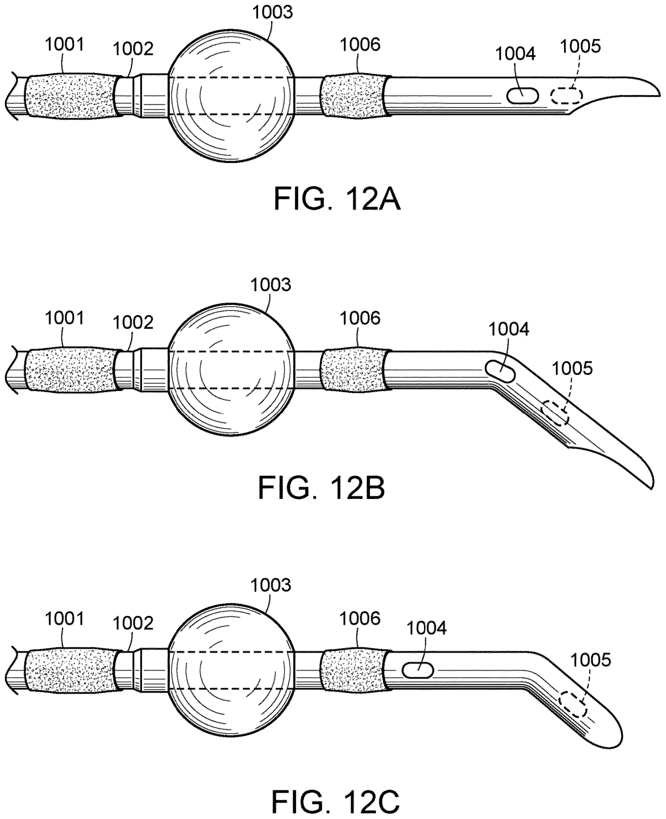

As shown in Figs. 12A-C, one embodiment of the invention shown in Figs. 6A-B with sleeve portions **1001** and **1006,** catheter body **1002,** retaining device **1003,** drainage eyelet **1004** and alternative instillation eyelet **1005** may have various shapes to the end that is inserted into the bladder. For example, Fig. 12 A shows a couvelaire tip, Fig. 12B shows a dufour tip and Fig. 12C shows a coude tip.

It is necessary for the fluid to be effluxed continuously at a basal rate to effect the continual washing of the periurethral space, where bacteria adhere, to prevent formation of biofilms and resulting bacterial infections. However, it is also contemplated that the fluid may be continuously effluxed from the semipermeable membrane(s) in a peristaltic wave action along the length of the catheter body in addition to the basal rate.

For the purposes of promoting an understanding of the principles of the invention, reference has been made to the preferred embodiments illustrated in the drawings, and specific language has been used to describe these embodiments. However, this specific language intends no limitation of the scope of the invention, and the invention should be construed to encompass all embodiments that would normally occur to one of ordinary skill in the art. The particular implementations shown and described herein are illustrative examples of the invention and are not intended to otherwise limit the scope of the invention in any way. For the sake of brevity, conventional aspects of the method (and components of the individual operating components of the method) may not be described in detail. Furthermore, the connecting lines, or connectors shown in the various figures presented are intended to represent exemplary functional relationships and/or physical or logical couplings between the various elements. It should be noted that many alternative or additional functional relationships, physical connections or logical connections might be present in a practical device. Moreover, no item or component is essential to the practice of the invention unless the element is specifically described as "essential" or "critical". Numerous modifications and adaptations will be readily apparent to those skilled in this art without departing from the scope of the present invention. It is noted that the scope of protection of the current invention is solely defined by the appended claims.

## Claims

1. A urinary catheter assembly comprising:
an elongate catheter body (401; 501; 601) having a proximal end (403; 503; 603) and a distal end (402; 502; 602);
a first sleeve portion (406; 506; 606) comprising a semipermeable membrane, wherein the first sleeve portion (406; 506; 606) is disposed on an outer surface of at least one portion of the catheter body (401; 501; 601); and
a first instillation lumen (410; 509; 607) at the proximal end (403; 503; 603) of the catheter body (401; 501; 601), wherein the first instillation lumen (410; 509; 607) is in fluid communication with the first sleeve portion (406; 506; 606);
**characterized in that** the urinary catheter assembly further comprises:
a pump in fluid communication with said first instillation lumen (410; 509; 607);
wherein the pump is operable to continuously move a first fluid through the first instillation lumen (410; 509; 607) to the first sleeve portion (406; 506; 606) and to continuously and circumferentially efflux the first fluid out of the semipermeable membrane of the first sleeve portion (406; 506; 606).

2. The urinary catheter assembly according to claim 1 wherein the first sleeve portion (406; 506; 606) is at a tip of the distal end (402; 502; 602) of the catheter body (401; 501; 601).

3. The urinary catheter assembly according to claim 1 or 2 further comprising a pressure and flow regulating valve operable to regulate a flow rate and a pressure of the first fluid effluxing through the first sleeve portion (406; 506; 606).

4. The urinary catheter assembly according to claim 3 wherein the pump comprises a pump tension device operable to regulate a flow rate and a pressure of the first fluid effluxing through the first sleeve portion (406; 506; 606).

5. The urinary catheter assembly according to claim 3 or 4 wherein the pump comprises an intravenous (IV) pump operable to operate at a continuous rate and operable to regulate a flow rate and a pressure of the first fluid effluxing through the first sleeve portion (406; 506; 606).

6. The urinary catheter assembly according to any one of claims 3 to 5 wherein the flow rate and the pressure of the first fluid effluxing through the first sleeve portion (406; 506; 606) is predetermined based on the material used for the semipermeable membrane of the first sleeve portion (406; 506; 606) and calculated based on a molecular weight cut off (MWCO) of the first fluid.

7. The urinary catheter assembly according to any one of the preceding claims wherein the pore size of the semipermeable membrane of the first sleeve portion (406; 506; 606) is predetermined and calculated based on a molecular weight cut off (MWCO) of the first fluid.

8. The urinary catheter assembly according to any one of the preceding claims further comprising:
a second sleeve portion (609) comprising a semipermeable membrane, wherein the second sleeve portion (609) is disposed on the outer surface of at least one portion of the catheter body (601);
a second instillation lumen (611) at the proximal end (603) of the catheter body (601), wherein the second instillation lumen (611) is in fluid communication with the second sleeve portion (609); and
a second pump in fluid communication with said second instillation lumen (611);
wherein the second pump is operable to continuously move a second fluid through the second instillation lumen (611) to the second sleeve portion (609) and to continuously and circumferentially efflux the second fluid out of the semipermeable membrane of the second sleeve portion (609).

9. The urinary catheter assembly according claim 8 further comprising a second pressure and flow regulating valve operable to regulate a flow rate and a pressure of the second fluid effluxing through the second sleeve portion (609).

10. The urinary catheter assembly according to claim 8 or 9 wherein the second pump comprises a pump tension device operable to regulate a flow rate and a pressure of the second fluid effluxing through the second sleeve portion.

11. The urinary catheter assembly according to any one of claims 8 to 10 wherein the second pump comprises an intravenous (IV) pump operable to operate at a continuous rate and operable to regulate a flow rate and a pressure of the second fluid effluxing through the second sleeve portion (609).

12. The urinary catheter assembly according to any one of claims 8 to 11 wherein the flow rate and the pressure of the second fluid effluxing through the second sleeve portion (609) is predetermined based on the material used for the semipermeable membrane of the second sleeve portion (609) and calculated based on a molecular weight cut off (MWCO) of the second fluid.

13. The urinary catheter assembly according to any one of claims 8 to 12 wherein the pore size of the semipermeable membrane of the second sleeve portion (609) is predetermined and calculated based on the molecular weight cut off (MWCO) of the second fluid.

14. The urinary catheter assembly according to any one of claims 8 to 13 further comprising a retaining mechanism towards the distal end (602) of the catheter body (601).

15. The urinary catheter assembly according to claim 14, wherein the first sleeve portion (606) is between the proximal end (603) of the catheter body (601) and the retaining mechanism.

16. The urinary catheter assembly according to claim 14 or 15, wherein the second sleeve portion (609) is between the distal end (602) of the catheter body (601) and the retaining mechanism.

17. The urinary catheter assembly according to any one of claims 14 to 16 wherein the first sleeve portion (606) is at the tip of the distal end (602) of the catheter body (601) and the second sleeve portion (609) is located between the proximal end (603) of the catheter body (601) and the retaining mechanism.

18. The urinary catheter assembly according to any one of the preceding claims further comprising a drainage lumen (404; 504; 604) and at least one drainage opening (405; 505; 605)at the distal end (402; 502; 602) of the catheter body (401; 501; 601), wherein the drainage lumen (404; 504; 604) extends through the catheter body (401; 501; 601) and is in fluid communication with the at least one drainage opening (405; 505; 605).

## Patentansprüche

1. Blasenkatheteraufbau, umfassend:
einen länglichen Katheterkörper (401; 501; 601) mit einem proximalen Ende (403; 503; 603) und einem distalen Ende (402; 502; 602);
einen ersten Hülsenabschnitt (406; 506; 606), umfassend eine halbdurchlässige Membran, wobei der erste Hülsenabschnitt (406; 506; 606) an einer Außenfläche von mindestens einem Abschnitt des Katheterkörpers (401; 501; 601) angeordnet ist; und
ein erstes Einträufelungslumen (410; 509; 607) an dem proximalen Ende (403; 503; 603) des Katheterkörpers (401; 501; 601), wobei das erste Einträufelungslumen (410; 509; 607) in Fluidverbindung mit dem ersten Hülsenabschnitt (406; 506; 606) steht; **dadurch gekennzeichnet, dass** der Blasenkatheteraufbau ferner Folgendes umfasst:
eine Pumpe in Fluidverbindung mit dem ersten Einträufelungslumen (410; 509; 607); wobei die Pumpe bedienbar ist, um kontinuierlich ein erstes Fluid durch das erste Einträufelungslumen (410; 509; 607) zum ersten Hülsenabschnitt (406; 506; 606) zu bewegen und das erste Fluid kontinuierlich und umfänglich aus der halbdurchlässigen Membran des ersten Hülsenabschnitts (406; 506; 606) auszulassen.

2. Blasenkatheteraufbau nach Anspruch 1, wobei sich der erste Hülsenabschnitt (406; 506; 606) an einer Spitze des distalen Endes (402; 502; 602) des Katheterkörpers (401; 501; 601) befindet.

3. Blasenkatheteraufbau nach Anspruch 1 oder 2, ferner umfassend ein den Druck und den Durchfluss regulierendes Ventil, das bedienbar ist, um eine Durchflussmenge und einen Druck des ersten Fluids zu regulieren, das durch den ersten Hülsenabschnitt (406; 506; 606) austritt.

4. Blasenkatheteraufbau nach Anspruch 3, wobei die Pumpe eine Pumpenspannvorrichtung umfasst, die bedienbar ist, um eine Durchflussmenge und einen Druck des ersten Fluids zu regulieren, das durch den ersten Hülsenabschnitt (406; 506; 606) austritt.

5. Blasenkatheteraufbau nach Anspruch 3 oder 4, wobei die Pumpe eine intravenöse (IV) Pumpe umfasst, die bedienbar ist, um mit einer kontinuierlichen Rate zu arbeiten, und die bedienbar ist, um eine Durchflussmenge und einen Druck des ersten Fluids zu regulieren, das durch den ersten Hülsenabschnitt (406; 506; 606) austritt.

6. Blasenkatheteraufbau nach einem der Ansprüche 3 bis 5, wobei die Durchflussmenge und der Druck des ersten Fluids, das durch den ersten Hülsenabschnitt (406; 506; 606) austritt, basierend auf dem Material vorbestimmt sind, das für die halbdurchlässige Membran des ersten Hülsenabschnitts (406; 506; 606) verwendet wird, und basierend auf einer Trenngrenze (Molecular Weight Cut Off, MWCO) des ersten Fluids berechnet werden.

7. Blasenkatheteraufbau nach einem der vorhergehenden Ansprüche, wobei die Porengröße der halbdurchlässigen Membran des ersten Hülsenabschnitts (406; 506; 606) basierend auf der Trenngrenze (MWCO) des ersten Fluids vorbestimmt und berechnet wird.

8. Blasenkatheteraufbau nach einem der vorhergehenden Ansprüche, ferner umfassend:
einen zweiten Hülsenabschnitt (609), umfassend eine halbdurchlässige Membran, wobei der zweite Hülsenabschnitt (609) an der Außenfläche von mindestens einem Abschnitt des Katheterkörpers (601) angeordnet ist,
ein zweites Einträufelungslumen (611) am proximalen Ende (603) des Katheterkörpers (601), wobei das zweite Einträufelungslumen (611) in Fluidverbindung mit dem zweiten Hülsenabschnitt (609) steht; und
eine zweite Pumpe in Fluidverbindung mit dem zweiten Einträufelungslumen (611);
wobei die zweite Pumpe bedienbar ist, um ein zweites Fluid kontinuierlich durch das zweite Einträufelungslumen (611) zum zweiten Hülsenabschnitt (609) zu bewegen und das zweite Fluid kontinuierlich und umfänglich aus der halbdurchlässigen Membran des zweiten Hülsenabschnitts (609) auszulassen.

9. Blasenkatheteraufbau nach Anspruch 8, ferner umfassend ein zweites, den Druck und den Durchfluss regulierendes Ventil, das bedienbar ist, um eine Durchflussmenge und einen Druck des zweiten Fluids zu regulieren, das durch den zweiten Hülsenabschnitt (609) austritt.

10. Blasenkatheteraufbau nach Anspruch 8 oder 9, wobei die zweite Pumpe eine Pumpenspannvorrichtung umfasst, die bedienbar ist, um eine Durchflussmenge und einen Druck des zweiten Fluids zu regulieren, das durch den zweiten Hülsenabschnitt austritt.

11. Blasenkatheteraufbau nach einem der Ansprüche 8 bis 10, wobei die zweite Pumpe eine intravenöse (IV) Pumpe umfasst, die bedienbar ist, um mit einer kontinuierlichen Rate zu arbeiten, und die bedienbar ist, um eine Durchflussmenge und einen Druck des zweiten Fluids zu regulieren, das durch den zweiten Hülsenabschnitt (609) austritt.

12. Blasenkatheteraufbau nach einem der Ansprüche 8 bis 11, wobei die Durchflussmenge und der Druck des zweiten Fluids, das durch den zweiten Hülsenabschnitt (609) austritt, basierend auf dem Material vorbestimmt sind, das für die halbdurchlässige Membran des zweiten Hülsenabschnitts (609) verwendet wird, und basierend auf einer Trenngrenze (MWCO) des zweiten Fluids berechnet werden.

13. Blasenkatheteraufbau nach einem der Ansprüche 8 bis 12, wobei die Porengröße der halbdurchlässigen Membran des zweiten Hülsenabschnitts (609) basierend auf der Trenngrenze (MWCO) des zweiten Fluids vorbestimmt und berechnet wird.

14. Blasenkatheteraufbau nach einem der Ansprüche 8 bis 13, ferner umfassend einen Haltemechanismus zum distalen Ende (602) des Katheterkörpers (601) hin.

15. Blasenkatheteraufbau nach Anspruch 14, wobei der erste Hülsenabschnitt (606) zwischen dem proximalen Ende (603) des Katheterkörpers (601) und dem Haltemechanismus liegt.

16. Blasenkatheteraufbau nach Anspruch 14 oder 15, wobei der zweite Hülsenabschnitt (609) zwischen dem distalen Ende (602) des Katheterkörpers (601) und dem Haltemechanismus liegt.

17. Blasenkatheteraufbau nach einem der Ansprüche 14 bis 16, wobei sich der erste Hülsenabschnitt (606) an der Spitze des distalen Endes (602) des Katheterkörpers (601) befindet und sich der zweite Hülsenabschnitt (609) zwischen dem proximalen Ende (603) des Katheterkörpers (601) und dem Haltemechanismus befindet.

18. Blasenkatheteraufbau nach einem der vorhergehenden Ansprüche, ferner umfassend ein Drainagelumen (404; 504; 604) und mindestens eine Drainageöffnung (405; 505; 605) am distalen Ende (402; 502; 602) des Katheterkörpers (401; 501; 601), wobei sich das Drainagelumen (404; 504; 604) durch den Katheterkörper (401; 501; 601) erstreckt und in Fluidverbindung mit der mindestens einen Drainageöffnung (405; 505; 605) steht.

## Revendications

1. Ensemble formant cathéter urinaire comprenant :
un corps de cathéter allongé (401 ; 501 ; 601) ayant une extrémité proximale (403 ; 503 ; 603) et une extrémité distale (402 ; 502 ; 602) ;
une première partie formant manchon (406 ; 506 ; 606) comprenant une membrane semi-perméable, dans lequel la première partie formant manchon (406 ; 506 ; 606) est disposée sur une surface extérieure d'au moins une partie du corps de cathéter (401 ; 501 ; 601) ; et une première lumière d'instillation (410 ; 509 ; 607) en l'extrémité proximale (403 ; 503 ; 603) du corps de cathéter (401 ; 501 ; 601), dans lequel la première lumière d'instillation (410 ; 509 ; 607) est en communication fluidique avec la première partie formant manchon (406 ; 506 ; 606) ;
**caractérisé en ce que** l'ensemble formant cathéter urinaire comprend en outre :
une pompe en communication fluidique avec ladite première lumière d'instillation (410 ; 509 ; 607) ;
dans lequel la pompe est exploitable afin de faire passer de façon continue un premier fluide à travers la première lumière d'instillation (410 ; 509 ; 607) vers la première partie formant manchon (406 ; 506 ; 606) et de faire s'écouler de façon continue et circonférentielle le premier fluide en dehors de la membrane semi-perméable de la première partie formant manchon (406 ; 506 ; 606).

2. Ensemble formant cathéter urinaire selon la revendication 1, dans lequel la première partie formant manchon (406 ; 506 ; 606) se trouve en une pointe de l'extrémité distale (402 ; 502 ; 602) du corps de cathéter (401 ; 501 ; 601).

3. Ensemble formant cathéter urinaire selon la revendication 1 ou 2 comprenant en outre une soupape de régulation de la pression et de l'écoulement exploitable afin de réguler un débit et une pression du premier fluide s'écoulant à travers la première partie formant manchon (406 ; 506 ; 606).

4. Ensemble formant cathéter urinaire selon la revendication 3, dans lequel la pompe comprend un dispositif de tension de pompe exploitable afin de réguler un débit et une pression du premier fluide s'écoulant à travers la première partie formant manchon (406 ; 506 ; 606).

5. Ensemble formant cathéter urinaire selon la revendication 3 ou 4, dans lequel la pompe comprend une pompe intraveineuse (IV) exploitable afin de fonctionner à un débit constant et exploitable afin de réguler un débit et une pression du premier fluide s'écoulant à travers la première partie formant manchon (406 ; 506 ; 606).

6. Ensemble formant cathéter urinaire selon l'une quelconque des revendications 3 à 5, dans lequel le débit et la pression du premier fluide s'écoulant à travers la première partie formant manchon (406 ; 506 ; 606) est prédéterminé en se fondant sur le matériau utilisé pour la membrane semi-perméable de la première partie formant manchon (406 ; 506 ; 606) et calculé en se fondant sur une perte de poids moléculaire (MWCO) du premier fluide.

7. Ensemble formant cathéter urinaire selon l'une quelconque des revendications précédentes, dans lequel la grosseur de pore de la membrane semi-perméable de la première partie formant manchon (406 ; 506 ; 606) est prédéterminée et calculée en se fondant sur une perte de poids moléculaire (MWCO) du premier fluide.

8. Ensemble formant cathéter urinaire selon l'une quelconque des revendications précédentes, comprenant en outre :
une deuxième partie formant manchon (609) comprenant une membrane semi-perméable, dans lequel la deuxième partie formant manchon (609) est disposée sur la surface extérieure d'au moins une partie du corps de cathéter (601) ;
une deuxième lumière d'instillation (611) en l'extrémité proximale (603) du corps de cathéter (601), dans lequel la deuxième lumière d'instillation (611) est en communication fluidique avec la deuxième partie formant manchon (609) ; et
une deuxième pompe en communication fluidique avec ladite deuxième lumière d'instillation (611) ;
dans lequel la deuxième pompe est exploitable afin de faire passer de façon continue un deuxième fluide à travers la deuxième lumière d'instillation (611) vers la deuxième partie formant manchon (609) et de faire s'écouler de façon continue et circonférentielle le deuxième fluide en dehors de la membrane semi-perméable de la deuxième partie formant manchon (609).

9. Ensemble formant cathéter urinaire selon la revendication 8 comprenant en outre une deuxième soupape de régulation de la pression et de l'écoulement exploitable afin de réguler un débit et une pression du deuxième fluide s'écoulant à travers la deuxième partie formant manchon (609).

10. Ensemble formant cathéter urinaire selon la revendication 8 ou 9, dans lequel la deuxième pompe comprend un dispositif de tension de pompe exploitable afin de réguler un débit et une pression du deuxième fluide s'écoulant à travers la deuxième partie formant manchon.

11. Ensemble formant cathéter urinaire selon l'une quelconque des revendications 8 à 10, dans lequel la deuxième pompe comprend une pompe intraveineuse (IV) exploitable afin de fonctionner à un débit constant et exploitable afin de réguler un débit et une pression du deuxième fluide s'écoulant à travers la deuxième partie formant manchon (609).

12. Ensemble formant cathéter urinaire selon l'une quelconque des revendications 8 à 11, dans lequel le débit et la pression du deuxième fluide s'écoulant à travers la deuxième partie formant manchon (609) est prédéterminé en se fondant sur le matériau utilisé pour la membrane semi-perméable de la deuxième partie formant manchon (609) et calculé en se fondant sur une perte de poids moléculaire (MWCO) du deuxième fluide.

13. Ensemble formant cathéter urinaire selon l'une quelconque des revendications 8 à 12, dans lequel la grosseur de pore de la membrane semi-perméable de la deuxième partie formant manchon (609) est prédéterminée et calculée en se fondant sur la perte de poids moléculaire (MWCO) du deuxième fluide.

14. Ensemble formant cathéter urinaire selon l'une quelconque des revendications 8 à 13, comprenant en outre un mécanisme de retenue en direction de l'extrémité distale (602) du corps de cathéter (601).

15. Ensemble formant cathéter urinaire selon la revendication 14, dans lequel la première partie formant manchon (606) se trouve entre l'extrémité proximale (603) du corps de cathéter (601) et le mécanisme de retenue.

16. Ensemble formant cathéter urinaire selon la revendication 14 ou 15, dans lequel la deuxième partie formant manchon (609) se trouve entre l'extrémité distale (602) du corps de cathéter (601) et le mécanisme de retenue.

17. Ensemble formant cathéter urinaire selon l'une quelconque des revendications 14 à 16, dans lequel la première partie formant manchon (606) se trouve à la pointe de l'extrémité distale (602) du corps de cathéter (601) et la deuxième partie formant manchon (609) se situe entre l'extrémité proximale (603) du corps de cathéter (601) et le mécanisme de retenue.

18. Ensemble formant cathéter urinaire selon l'une quelconque des revendications précédentes, comprenant en outre une lumière de drainage (404 ; 504 ; 604) et au moins un orifice de drainage (405 ; 505 ; 605) en l'extrémité distale (402 ; 502 ; 602) du corps de cathéter (401 ; 501 ; 601), dans lequel la lumière de drainage (404 ; 504 ; 604) s'étend à travers le corps de cathéter (401 ; 501 ; 601) et est en communication fluidique avec l'au moins un orifice de drainage (405 ; 505 ; 605).
